# EUROPEAN PATENT APPLICATION

(11) **EP 1 050 317 A1**
(43) Date of publication of application: **08.11.2000**
(21) Application number: 00109487.9
(22) Date of filing: 04.05.2000
(51) Int. Cl.: A61M 25/04

(54) **Locking device for a catheter and a catheter**

(30) Priority: 07.05.1999 IT TO990373
(71) Applicant: Karbix Establishment, 9490 Vaduz (LI)
(72) Inventor: Ferrando, Ugo, 10128 Torino (IT)
(74) Representative: Lotti, Giorgio

(57) **Abstract**

A locking device (1) for a catheter (2) provided with two bearing rings (5) which are substantially movable and placed along a stem (6) of the catheter (2), and two helical spacer elements which are wound around the stem (6) and present respective end portions (8)(9) integral to said rings (5).

## Description

The present invention relates to a locking device for a catheter.

European Patent Application No. 0 113 489, discloses a well known locking device which is able to lock a catheter placed inside a duct in a fixed position, and comprises a number of pair of tongues mounted integral to a lengthened stem of the catheter.

The tongues in each pair of tongues radially overhang towards the outside of the catheter of the stem, and are placed on opposite sides of the stem. The tongues in each pair of tongues are secured to the stem by means of a heat-sealed bearing ring.

The above-described locking device, even though it is reliable and easy-to-use, presents some drawbacks because, at first, the separation of the duct from the stem is only possible by using a relatively large number of pairs of tongues, and the consequence of this is that a large number of heat-sealed bearing rings must be used.

Furthermore, as the number of tongues can be high to the detriment of both the cost and the simplicity, the pressure exerted by the tongues against the duct causes a relatively high trophical pain in the duct of the patient.

In the end, bearing in mind that each ring is heat-sealed to the stem, no eventual adjustment - either of the duct diameter or of the duct length engaged by the locking device - can be carried out by means of the same locking device.

It is an aim of the present invention to provide a locking device for a catheter device capable of overcoming the above-mentioned drawbacks.

According to the present invention, a locking device for a catheter is provided and comprises at least one bearing ring placed along the stem of the catheter, and is characterised by the fact that it further comprises helical spacer means which are wound around the said stem and are provided with at least one end portion integral to said ring.

The present invention further provides a catheter.

According to the present invention, a catheter is provided and comprises a stem and at least one locking device, which is placed along the stem and comprises at least one bearing ring placed along said stem; the catheter is characterised by the fact that said locking device comprises helical spacer means which are wound around the said stem and are provided with at least one end portion integral to said ring.

A preferred and non limiting embodiment of the invention will now be described with reference to the accompanying drawings, in which:
FIG. 1 shows a preferred embodiment of a locking device for a catheter according to the present invention; and
FIG. 2 shows a preferred embodiment of a catheter provided with the locking device shown in figure 1.

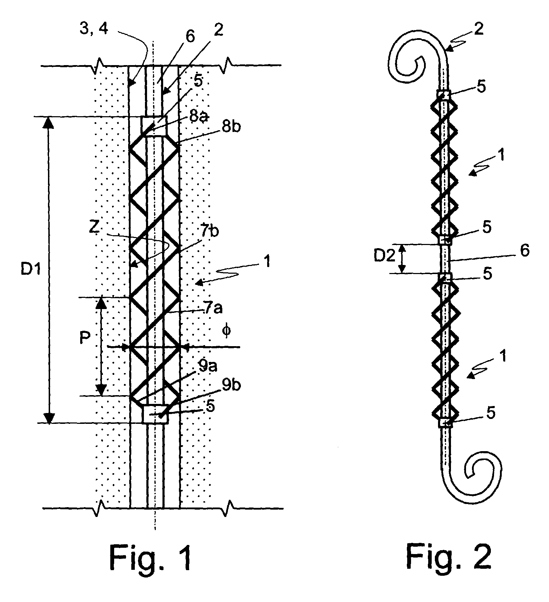

With reference initially to FIG. 1, the number 1 indicates, in its entirety, a locking device for a catheter 2 placed inside a duct 3.

The locking device 1 is able to lock the catheter 2 in relation to the duct 3 co-operating with an internal surface 4 of the duct 3, and comprises two bearing rings 5 adjustably placed along a stem 6 of the catheter 2, and two continuous elements 7 which are helically wound around the stem 6 with a respective adjustable pitch P. The two elements 7 are able to arrange themselves in contact along a contact area Z of the surface 4 so as to keep the stem 6 apart from the surface 4 and so as to lock the stem 4 as regards the surface 6 at the same time.

The two elements 7 are placed along relevant opposite sides of the stem 6, and each of the elements 7 has respective opposite end portions 8 and 9 integral to the rings 5: more particularly, identifying the two elements 7 and the relevant end portions 8 and 9 with the letters "a" and "b", the end portions 8a and 8b are mounted on the same ring 5 spaced at 180° to each other, and the end portions 9a and 9b are mounted on the other ring 5 spaced of 180° at to each other.

The rings 5 are mounted on the stem 6, and are able to slide in relation with each other along the stem 6. The rings 5 are placed at a regulating distance D1 from one another so that, by moving at least one of the rings 5 in relation to the other ring 5, it is possible both to change the pitch P of the elements 7, increasing or decreasing the longitudinal length of the contact area Z, and to increase or decrease an external diameter ⌀ of the device 1. Furthermore, the rings 5 are able to rotate in relation to each other, and the rotation of one of the two rings 5 causes either an increase or a decrease in the diameter ⌀ of the device 1 without any appreciable modification in the length of the contact area Z.

The mutual movement of rotation and the mutual movement of axial shift of the rings 5 can be combined in different ways so that it is easy to vary the configuration of the device 1 to adapt it to any operating requirements.

According to an embodiment of the device 1 which is not shown, one of the two elements 7 can be omitted, but the remaining element 7 will permit all the aforementioned settings.

Figure 1 shows a catheter 2 which comprises a stem 6 and two locking devices 1 as described above, which are placed in relation to each other at a regulating distance D2 so that the contact zone Z along the duct 3 can be longitudinally lengthened.

The above-described device 1 and catheter 2 are advantageously useful in the urological field, and are able to be installed along a ureter, and from the above it is clear that the locking device 1 permits a better distribution of the pressure of the catheter 2 against the surface 4 and reduces trophical pain in the surface 4.

Furthermore, the helical shape of the elements 77 permits a better drainage of the urine even though the presence of broken renal calculus after extra corporeal shockwave litotripsy or endoscopic treatments.

While a specific embodiment of the invention has been disclosed, it is to be understood that such disclosure has been merely for the purpose of illustration and that the invention is not to be limited in any manner thereby. Various modifications will be apparent to those skilled in the art in view of the foregoing example. The scope of the invention is to be limited only by the appended claims.

## Claims

1. A locking device (1) for a catheter (2) comprising at least one bearing ring (5) placed along the stem (6) of the catheter (2), and is characterised by the fact that it further comprises helical spacer means which are wound around the said stem (6) and are provided with at least one end portion (8)(9) integral to said ring (5).

2. A locking device as claimed in claim 1, characterised by the fact that it comprises two bearing rings (5) placed along the stem (6) at a first adjustable distance from one another; the said helical means (7) comprising two end portions (8)(9) which are integral to said rings (5) and present a selective pitch (P) which is changeable along the said stem (6).

3. A locking device as claimed in claim 2, characterised by the fact that the said helical means (7) present an external diameter (⌀) whose overall dimensions are selectively changeable.

4. A locking device as claimed in claim 1, claim 2, or claim 3, characterised by the fact that the said helical means (7) comprise a continuous element (7) which is helically wound around the said stem (6).

5. A locking device as claimed in claim 4, characterised by the fact that the said helical means (7) comprise two continuous elements (7) which are helically wound around the said stem (6).

6. A locking device as claimed in claim 5, characterised by the fact that the said two continuous elements (7) develop along opposing positions along the said stem (6).

7. A catheter (2) comprising a stem (6) and at least one locking device, which is placed along the stem (6) and comprises at least one bearing ring (5) placed along said stem (6); the catheter being characterised by the fact that said locking device comprises helical spacer (7) means which are wound around the said stem (6) and are provided with at least one end portion (8)(9) integral to said ring (5).

8. A catheter as claimed in claim 7, characterised by the fact that it comprises at least two locking devices (1) placed at a second adjustable distance (D2) from one another.
